# EUROPEAN PATENT APPLICATION

(11) **EP 4 431 531 A1**
(43) Date of publication of application: **18.09.2024**
(21) Application number: 22893251.3
(22) Date of filing: 11.11.2022
(51) Int. Cl.: C08B 37/08, C08B 37/00, C08L 5/08, A61K 31/167

(54) **METHOD FOR PREPARING COMPOSITION CONTAINING HYALURONIC ACID CROSSLINKED PRODUCT**

(30) Priority: 12.11.2021 KR 20210155528
(71) Applicant: Yoo Young Pharm Co., Ltd., Seoul 06687 (KR)
(72) Inventor: AH, Young Chang, Suwon-si Gyeonggi-do 16514 (KR); CHO, Wan Jin, Bucheon-si Gyeonggi-do 14579 (KR); YANG, Jin Hyo, Suwon-si Gyeonggi-do 16369 (KR); KIM, Jeong Eun, Seoul 08555 (KR)
(74) Representative: Louis Pöhlau Lohrentz
(86) International application number: PCT/KR2022/017707
(87) International publication number: WO 2023/085832

(57) **Abstract**

Disclosed is a method for preparing a composition containing a hyaluronic acid crosslinked product.

## Description

### Technical Field

The present invention relates to a method for producing a composition containing crosslinked hyaluronic acid, which is capable of controlling the physical properties of crosslinked hyaluronic acid regardless of the composition of a mixture containing hyaluronic acid or a salt thereof and a chemical crosslinking agent in the process of producing a water-insoluble gel-type crosslinked hyaluronic acid by chemically modifying hyaluronic acid or a salt thereof.

### Background Art

Hyaluronic acid (HA) refers to any known hyaluronic acid raw materials alone or in combination, such as hyaluronic acid, its salts, or its precursors. Hyaluronic acid is a natural polymer material that has excellent biocompatibility and is widely used in various applications, including cosmetic or medical applications. Naturally occurring hyaluronic acid is found mainly in the extracellular matrix and plays a role in stabilizing tissue structure by mixing with fiber and collagen to form matrix fluid. This is a substance found in the tissues of all vertebrates, and is non-immunogenic, making it an ideal chemical substance for use as a component for tissue repair purposes.

Hyaluronic acid is a substance that is widely applied in the fields of cosmetics, food, medical devices, and pharmaceuticals. In addition, a water-insoluble gel-type crosslinked hyaluronic acid produced by chemically crosslinking hyaluronic acid is characterized in that the physical properties thereof are easy to control and the period during which hyaluronic acid remains in the body may be extended ranging from within a week or less to several months or even about one year. These characteristics of crosslinked hyaluronic acid are the reason why the crosslinked hyaluronic acid is used as biomaterials for tissue repair that require long-term *in vivo* retention, scaffolds for drug delivery, or osteoarthritis therapeutic agents. The most widely used chemical crosslinking agents for converting hyaluronic acid into crosslinked hyaluronic acid include bisepoxides, such as 1,4-butanediol diglycidyl ether (BDDE), and divinyl sulfone (DVS). In order to convert hyaluronic acid into crosslinked hyaluronic acid using the above chemical crosslinking agent, the hydrogen ion concentration (pH) of a mixture containing the hyaluronic acid should be 13 or higher. Therefore, hyaluronic acid and a chemical crosslinking agent (BDDE or DVS) should be mixed with a strong basic solvent so that crosslinked hyaluronic acid may be produced.

The physical properties and *in vivo* duration of crosslinked hyaluronic acid are determined by the composition of a hyaluronic acid crosslinking mixture, which is prepared by mixing hyaluronic acid, a chemical crosslinking agent, and a strong basic solvent, and the specific conditions of the crosslinking process for producing crosslinked hyaluronic acid. Depending on the composition of the hyaluronic acid crosslinking mixture and the specific conditions of the crosslinking process, the degree of modification (Equation 1), which is the bonding ratio between hyaluronic acid and the chemical crosslinking agent, and the degree of crosslinking, which is the ratio of crosslinks formed by chemical crosslinking (Equation 2), vary, and ultimately the properties of crosslinked hyaluronic acid are represented by the swelling degree of the crosslinked hyaluronic acid (Equation 3). Degree of modification = Number of repeating units combined with crosslinking agent / number of repeating units in water-soluble polymer x 100 Degree of crosslinking = Number of repeating units forming crosslinks / number of repeating units in water-soluble polymer x 100 Swelling degree = Dry weight of water-insoluble gel / weight of swollen water-insoluble gel

The method of controlling the composition of the hyaluronic acid crosslinking mixture or controlling the specific conditions of the crosslinking process to control the swelling degree of the crosslinked hyaluronic acid has two problems.

The first problem is to develop and manage the composition of the crosslinking mixture. Since a crosslinking mixture with a specific composition should be developed according to the physical properties of the desired crosslinked hyaluronic acid, if the target physical properties of the crosslinked hyaluronic acid are changed, the composition of the crosslinking mixture should be changed. Although a technology for removing water during the crosslinking process to change the physical properties of crosslinked hyaluronic acid without changing the composition of a crosslinking mixture has been developed (Korean Patent No. 1316401), the conditions for removing water are vacuum conditions that are difficult to precisely control, and the problem of controlling the physical properties of the final crosslinked hyaluronic acid by the composition of the crosslinking mixture still remains. In addition, a technology has been proposed in which the composition of the crosslinking mixture remains the same, but first crosslinking and additional mixing are performed after preparation of the crosslinking mixture, and then second crosslinking is performed (Korean Patent Application Publication No. 2019-0059609). In this technology, the composition of the prepared crosslinking mixture still determines the physical properties of the crosslinked hyaluronic acid. In addition, the molecular weight of hyaluronic acid should be hundreds of thousands or more to enable chemical crosslinking under strong basic conditions. However, hyaluronic acid with a molecular weight of hundreds of thousands or more, when mixed with water, exhibits high viscosity, making mixing difficult, and when the concentration of hyaluronic acid is low, it is difficult to form a crosslinked product. Due to this problem, the hyaluronic acid content in the crosslinking mixture is usually within 10 to 30%. In other words, there are limits to changing the composition of the crosslinking mixture.

The second problem is that there are limits to controlling the specific conditions of the crosslinking process. In order to form a crosslink between hyaluronic acid and a chemical crosslinking agent, a strong basic solvent is required. The strong basic solvent exhibits the effect of lowering the molecular weight of hyaluronic acid by decomposing hyaluronic acid, and the decomposition of hyaluronic acid by the strong basic solvent increases as the temperature increases and as the contact time between the strong basic solvent and hyaluronic acid increases. In other words, in order to produce a crosslinked hyaluronic acid with a low degree of swelling by using a crosslinking mixture with the same composition, it is necessary to increase the crosslinking temperature or the crosslinking time. However, if the crosslinking process is performed at a certain temperature or higher or for a certain period of time or longer, a problem arises in that the swelling degree of the crosslinked hyaluronic acid increases rather than decreases, or if the crosslinking mixture is stored at a high temperature for an excessively long period of time, a problem arises in that serious browning occurs due to modification of the chemical structure of hyaluronic acid in the crosslinking mixture (Korean Patent Application Publication No. 10-2021-0009916), and ultimately, crosslinked hyaluronic acid cannot be formed. In conclusion, there is a limit to controlling the physical properties of crosslinked hyaluronic acid, represented by the degree of swelling, only by controlling the crosslinking process.

In addition, the process of producing crosslinked hyaluronic acid includes a purification process of removing impurities including a chemical crosslinking agent added to produce crosslinked hyaluronic acid. Examples of this purification process include a purification process using water and a purification process using an organic solvent compatible with water. Among these processes, in the case of the purification process using water, crosslinked hyaluronic acid swells during the purification process, and if this swelling is not controlled, the target hyaluronic acid content in the crosslinked hyaluronic acid cannot be achieved. For example, in order to achieve the target hyaluronic acid content in crosslinked hyaluronic acid of 2 wt%, that is, 20 mg/g, the swelling degree according to Equation 3 above should be maintained at 50-fold or less. However, crosslinked hyaluronic acid with low elastic modulus may also show a swelling degree of 100-fold or more. To maintain the hyaluronic acid content at 2 wt% while having low elastic modulus, purification using a semi-permeable membrane is essential. If purification using a bag made of a membrane is set as a purification process for purifying crosslinked hyaluronic acid, there are disadvantages in that the time required for the purification process is longer, ranging from several days to 30 days, and is cumbersome to insert and withdraw crosslinked hyaluronic acid from a bag made of a membrane. Of course, technologies for increasing the efficiency of the crosslinked hyaluronic acid purification process (Korean Patent No. 1922711) have been developed, but they are technologies for suppressing the swelling of crosslinked hyaluronic acid using osmotic pressure or for performing the purification process by trapping crosslinked hyaluronic acid within a mesh, and have limitations in suppressing the swelling of crosslinked hyaluronic acid during the purification process.

### [Prior Art Documents]

### [Patent Documents]

(Patent Document 1) Korean Patent No. 1316401
(Patent Document 2) Korean Patent Application Publication No. 2019-0059609
(Patent Document 3) Korean Patent Application Publication No. 10-2021-0009916
(Patent Document 4) Korean Patent No. 1922711

### DISCLOSURE

### Technical Problem

The present inventors have sought to develop a production process that may easily control the physical properties of crosslinked hyaluronic acid and may produce a crosslinked hyaluronic acid-containing composition having a hyaluronic acid content within a wide range using the same crosslinked hyaluronic acid by suppressing the swelling degree of the crosslinked hyaluronic acid to the greatest possible extent during the purification process.

### Technical Solution

The present inventors have sought to control the physical properties of crosslinked hyaluronic acid by introducing a physical property control process for a crosslinked hyaluronic acid that has undergone a crosslinking process in order to control the physical properties of the crosslinked hyaluronic acid regardless of the composition of a crosslinking mixture and crosslinking conditions. The physical property control process has achieved its purpose by using a basic aqueous solution. In addition, the present inventors have introduced a method of drying crosslinked hyaluronic acid as a new means for suppressing the swelling degree of the crosslinked hyaluronic acid during the purification process.

### Advantageous Effects

The method for producing a crosslinked hyaluronic acid-containing composition according to the present invention is capable of variously changing the physical properties of crosslinked hyaluronic acid by post-treating the crosslinked hyaluronic acid even if the composition of a crosslinking mixture and the specific conditions of the crosslinking process are kept the same. Thus, no efforts need to be made to change the composition of the crosslinking mixture and the specific conditions of the crosslinking process to produce a target crosslinked hyaluronic acid or a composition containing a crosslinked hyaluronic acid. In addition, the swelling of crosslinked hyaluronic acid that occurs during the purification process may be effectively suppressed without using a physical structure such as a bag, made of a semi-permeable membrane, or a pressure vessel. As the swelling of the crosslinked hyaluronic acid is effectively suppressed and the efficiency of removing impurities from the crosslinked hyaluronic acid is maximized, the cost of the purification process is reduced and it is easy to control the hyaluronic acid content in the composition containing the crosslinked hyaluronic acid.

Effects of the present invention are not limited to the effects mentioned above, and various effects may be included within the scope apparent to those skilled in the art from the following description.

### Brief Description of Drawings

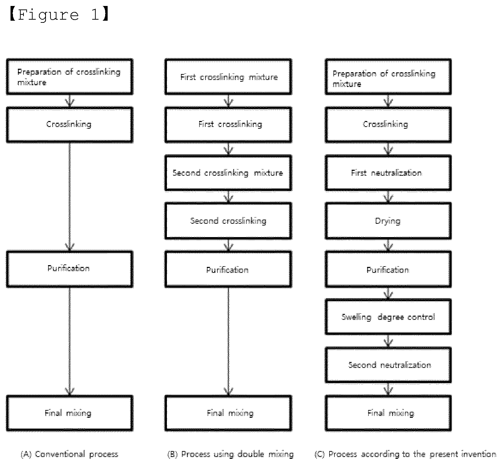

FIG. 1 shows a comparison between processes for producing a composition containing crosslinked hyaluronic acid.

### Best Mode

Hereinafter, the present specification will be described in more detail.

This is explained in detail as follows. The terms used in the present specification are currently widely used general terms selected in consideration of their functions in the present invention, but they may change depending on the intents of those skilled in the art, precedents, or the advents of new technology. Additionally, in certain cases, there may be terms arbitrarily selected by the applicant, and in this case, their meanings are described in a corresponding description part of the present invention. Accordingly, terms used in the present invention should be defined based on the meaning of the term and the entire contents of the present invention, rather than the simple term name.

Unless otherwise defined, all terms used herein, including technical or scientific terms, have the same meanings as commonly understood by those skilled in the art to which the present invention pertains. Terms such as those used in general and defined in dictionaries should be interpreted as having meanings identical to those specified in the context of related technology. Unless definitely defined in the present application, the terms should not be interpreted as having ideal or excessively formative meanings.

A numerical range includes numerical values defined in the range. Every maximum numerical limitation given throughout the present specification includes every lower numerical limitation, as if such lower numerical limitations were expressly written herein. Every minimum numerical limitation given throughout the present specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Meanwhile, each description and embodiment disclosed in the present invention may also be applied to other descriptions and embodiments. That is, all combinations of the various elements disclosed in the present invention fall within the scope of the present invention. Further, the scope of the present invention cannot be construed as being limited by the specific descriptions described below.

Expressions such as "comprising" or "containing" as used in the present specification should be understood as open-ended terms that imply the possibility of including other embodiments, unless otherwise mentioned in the phrase or sentence in which the expressions are contained.

The present inventors have found that the physical properties of crosslinked hyaluronic acid may be easily controlled and a crosslinked hyaluronic acid-containing composition having a hyaluronic acid content within a wide range may be produced using the same crosslinked hyaluronic acid by suppressing the swelling degree of the crosslinked hyaluronic acid to the greatest possible extent during the purification process, thereby completing the present invention.

Hereinafter, the present invention will be described in detail.

### Method for producing a composition containing crosslinked hyaluronic acid

The present invention discloses a method for producing a composition containing crosslinked hyaluronic acid, which is produced by the following steps (S1) to (S6):
(S1) a pre-crosslinking mixing process of mixing hyaluronic acid or a salt thereof, a chemical crosslinking agent, and a basic substance-containing aqueous solution having a hydrogen ion concentration (pH) of 12 to 14;
(S2) a crosslinking process of forming crosslinks;
(S3) a neutralization process of mixing a crosslinked hyaluronic acid, produced through the crosslinking process, with an aqueous solution containing an acidic substance, thus producing a crosslinked hyaluronic acid having a hydrogen ion concentration (pH) of 9 or less;
(S4) a drying process of removing water from a neutralized mixture prepared through (S3);
(S5) a washing process of removing water-soluble impurities from a hyaluronic acid gel by immersing the dried crosslinked hyaluronic acid, prepared through (S4), in a washing solution, which is an aqueous solution adjusted to an osmotic pressure of 0 to 600 mOsmol/kg using an osmotic pressure adjusting agent; and
(S6) a swelling control process of mixing the crosslinked hyaluronic acid, purified through (S5), with a basic substance or an aqueous solution containing a basic substance and an osmotic pressure adjusting agent.

In the present invention, the salt of hyaluronic acid may be sodium hyaluronate, without being limited thereto.

In the present invention, the following (S7) may be further included after (S6), without being limited thereto:
(S7) a final mixing process of mixing the crosslinked hyaluronic acid, whose swelling has been controlled through (S6), with an acidic substance or an aqueous solution containing an acidic substance, an osmotic pressure adjusting agent, an antioxidant additive, and a pain-relieving additive.

In the present invention, (S4) may be a drying process of removing water from the neutralized mixture prepared through (S3), thus producing a dried hyaluronic acid gel having a water content of 10% or less of the weight after drying, without being limited thereto.

In the present invention, (S4) may include a drying process of evaporating water while circulating dry air, and a drying process of removing water by immersing the neutralized crosslinked hyaluronic acid in an organic solvent, and then removing water and the organic solvent while circulating dry air, without being limited thereto.

In the present invention, the organic solvent may be at least one selected from the group consisting of methanol, ethanol, propanol, 1,2-butanediol, 1,3-butanediol, 1,5-pentanediol, 1,2-hexanediol, acetone, acetaldehyde, acetonitrile, tetrahydrofuran, glycerin, and tetraethylene glycol, without being limited thereto.

In the present invention, the crosslinking agent may be a bisepoxide-based crosslinking agent having at least two epoxy functional groups, or a divinyl sulfone-based crosslinking agent. Specifically, the crosslinking agent may be at least one selected from the group consisting of 1,4-butandiol diglycidyl ether, ethylene glycol diglycidyl ether, 1,6-hexanediol diglycidyl ether, propylene glycol diglycidyl ether, polyethylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, trimethlypropane polyglycidyl ether, 1,2-bis(2,3-epoxypropoxy)ethylene, pentaerythritol polyglycidyl ether, and sorbitol polyglycidyl ether, without being limited thereto.

In the present invention, the basic substance in (S1) and (S6) is a metal hydroxide, and refers to hydroxides of metals found in groups 1, 2, and 3 of the periodic table of elements. Specifically, the basic substance may be at least one selected from the group consisting of lithium hydroxide, sodium hydroxide, potassium hydroxide, calcium hydroxide, and aluminum hydroxide, without being limited thereto.

In the present invention, the acidic substance is an aqueous solution containing a substance that creates a pH of 6.0 or less when dissolved in water at a concentration of 0.1 mol/L. Specifically, the aqueous acidic substance solution may be at least one selected from the group consisting of an aqueous phosphoric acid solution, an aqueous acetic acid solution, an aqueous hydrochloric acid solution, an aqueous nitric acid solution, an aqueous sulfuric acid solution, an aqueous boric acid solution, an aqueous diethyl barbituric acid solution, sodium phosphate monobasic or sodium phosphate monobasic hydrate, potassium phosphate monobasic or potassium phosphate monobasic hydrate, ammonium phosphate monobasic or ammonium phosphate monobasic hydrate, and sodium acetate or sodium acetate hydrate, without being limited thereto.

In the present invention, (S6) may be a swelling control process of mixing the crosslinked hyaluronic acid, purified through (S5), with a basic aqueous solution so that the hydrogen ion concentration (pH) is adjusted to 11.4 to 12.0, without being limited thereto.

In the present invention, (S7) may be a final mixing process of mixing the crosslinked hyaluronic acid, whose physical properties have been controlled through (S6), with an acidic substance or an aqueous solution containing an acidic substance, an osmotic pressure adjusting agent, a viscosity-controlling additive, and a pain-relieving additive, thus producing a composition having a hydrogen ion concentration (pH) of 5 to 9 and a hyaluronic acid or salt content of 0.5 to 5 wt%, without being limited thereto.

In the present invention, the osmotic pressure adjusting agent may be at least one selected from the group consisting of sodium chloride (NaCl), potassium chloride (KCl), calcium chloride (CaCl₂), and magnesium chloride (MgCl₂), without being limited thereto.

In the present invention, the pain-relieving additive may be any one or more selected from the group consisting of lidocaine or a salt thereof and bupivacaine or a salt thereof, without being limited thereto.

In the present invention, the antioxidant additive may be at least one selected from the group consisting of ascorbic acid or a derivative thereof, glutathione, lipoic acid or a derivative thereof, uric acid or a derivative thereof, tocopherol, and mannitol, without being limited thereto.

Hereinafter, the present invention will be described in more detail by way of examples, but the present invention is not limited to the examples.

### Examples

Typically, in order to produce crosslinked hyaluronic acid, a crosslinking mixture preparation process, a crosslinking process, and a purification process are performed. In order to find an effective method for changing the physical properties of the crosslinked hyaluronic acid obtained through the crosslinking process, the present inventors selected heat treatment, treatment in a basic solution after purification, drying of neutralized crosslinked hyaluronic acid, and then treatment of the crosslinked hyaluronic acid in a basic solution, as methods for controlling the physical properties of crosslinked hyaluronic acid, and conducted experiments using the selected methods.

In order to evaluate the effect of heat treatment, the crosslinked hyaluronic acid subjected to the purification process was stored at different temperatures of 80°C and 100°C, and changes in the swelling degree of the crosslinked hyaluronic acid depending on storage time and whether the crosslinked hyaluronic acid maintained its shape were observed. Treatment in a basic solution was performed by immersing the purified crosslinked acid in a basic solution having a pH of 11 to 13.5, and changes in swelling degree of the crosslinked hyaluronic acid and whether crosslinked hyaluronic acid maintained its shape were observed. Although it could be confirmed that the swelling degree of the crosslinked hyaluronic acid was increased to a certain extent by these two post-treatment methods, it was not possible to control the swelling of the crosslinked hyaluronic acid that occurred during the purification process. As a result, it could be seen that these methods can be applied in situations where the crosslinking mixture or the crosslinking process is controlled so that the swelling degree that occurred during the purification process becomes equal to or less than the desired swelling degree.

In order to overcome the problems with the above-mentioned two methods of controlling the physical properties of crosslinked hyaluronic acid, a method of drying and then purifying the crosslinked hyaluronic acid was designed. In order to dry the crosslinked hyaluronic acid, the pH of the crosslinked hyaluronic acid produced through the crosslinking process should be adjusted. It could be seen that, when drying was performed at a pH higher than 9, the color of the crosslinked hyaluronic acid changed to brown, and when the dried crosslinked hyaluronic acid obtained through drying entered water, it was converted into a solution rather than a crosslinked hyaluronic acid. Thus, neutralization was essential to lower the pH of the crosslinked hyaluronic acid obtained through the crosslinking process, and the pH of the crosslinked hyaluronic acid was adjusted to 9 or less by neutralization. Drying was performed at a pH adjusted to 9 or less so that the water content in the dried crosslinked hyaluronic acid was 10% or less. The crosslinked hyaluronic acid dried in this way showed a swelling degree of about 10 to 32-fold in a washing solution whose osmotic pressure was adjusted to 280 to 300 mOsmol/kg. On the other hand, the crosslinked hyaluronic acid not subjected to the drying process showed a swelling degree of 32 to 200-fold in the washing solution whose osmotic pressure was adjusted to 280 to 300 mOsmol/kg. In particular, the crosslinked hyaluronic acid not subjected to the drying process showed a swelling degree of 199-fold in the washing solution, but when the crosslinked hyaluronic acid subjected to the drying process showed a swelling degree of 31.5-fold when immersed in the washing solution. These results show that a crosslinked hyaluronic acid capable of producing a composition having a hyaluronic acid content of up to 0.5 wt% is converted into a crosslinked hyaluronic acid capable of producing a composition having a hyaluronic acid content of up to 3.2% by weight through the drying process. This indicates that the hyaluronic acid content can be increased by 6.4 times by introducing the process of drying the crosslinked hyaluronic acid.

### [Comparative Examples 1 to 21 and Examples 1 to 21]

### 1. Mixing Process Before Crosslinking

A crosslinking mixture was prepared by mixing sodium hyaluronate (NaHA) having an intrinsic viscosity of 1.8 m³/kg, 1,4-butanediol diglycidyl ether (BDDE), and a 1% NaOH aqueous solution (pH = 13.4). Sodium hyaluronate was added in an amount of 10 to 25 wt% based on the total weight of the crosslinking mixture, and the crosslinking agent BDDE was adjusted to 3 to 5 mol% relative to the repeating units of hyaluronic acid. The amounts of sodium hyaluronate, BDDE, and 1% NaOH aqueous solution added are shown in Tables 1 and 2 below.

### 2. Crosslinking Process

After the preparation of the crosslinking mixture was completed, a crosslinking process was performed by storing the crosslinking mixture in a constant-temperature bath at 25°C for 24 hours.

### 3. Neutralization Process

A neutralization process was performed by immersing the crosslinked product, produced through the crosslinking process, in 100 g of an aqueous sodium phosphate monobasic solution containing 2.5 wt% of sodium phosphate monobasic (NaH₂PO₄) so that the aqueous sodium phosphate monobasic solution was completely absorbed into the crosslinked hyaluronic acid. The neutralization process was to neutralize strong basic substances present in the crosslinked hyaluronic acid composition obtained through the crosslinking process, and the pH of the obtained crosslinked hyaluronic acid composition was adjusted to 9 or less through the neutralization process.

### 4. Drying Process

After the neutralization process was completed, half of the neutralized crosslinked hyaluronic acid was immersed in 1 L of an isotonic solution (physiological saline) for 2 days for the purpose of measuring the swelling degree of the crosslinked hyaluronic acid, which was subjected to only the neutralization process after the crosslinking process, in the isotonic solution. The results of measuring the swelling degree in the isotonic solution after the neutralization process were classified as Comparative Examples 1 to 21 and summarized in Table 1 below.

The remaining half of the crosslinked hyaluronic acid was subjected to the drying process of removing water from the crosslinked product by storing the same in a convection oven at 60°C for 24 hours. In this case, the drying process was performed so that the water content was 10% or less of the weight after drying.

### 5. Washing in Isotonic Solution and Measurement of Swelling Degree

After the drying process was completed, the weight of the dried crosslinked hyaluronic acid was measured, and the dried hyaluronic acid was added to 1 L of an isotonic solution and purified by swelling until no increase in the swollen weight was observed. In addition, purification was completed by exchanging 1 L of the isotonic solution twice at 4-hour intervals, and the weight of the purified crosslinked hyaluronic acid was measured. The test procedure and measurement results for the crosslinked hyaluronic acid composition that was dried after neutralization were classified as Examples 1 to 21 and summarized in Table 2 below.

"Equation 4" below was applied to calculate the solid content in the dried crosslinked hyaluronic acid, and "Equation 5" below was applied to calculate the swelling degree of the crosslinked hyaluronic acid. Solid content (%) = Theoretical weight (g) of solids in HA gel / weight (g) of dried HA gel x 100 Swelling degree (fold) = swollen weight (g) of HA gel / theoretical content (g) of HA in HA gel

In Comparative Examples 1 to 21, when only the "neutralization process" was performed, the swelling degree was found to be 51.8 to 199.1-fold. This swelling degree is a swelling degree that cannot achieve a hyaluronic acid content of 2 wt% or more, which is most often applied in compositions containing crosslinked hyaluronic acid. If the swelling degree of the crosslinked hyaluronic acid during the purification process becomes higher than the swelling degree appropriate for the target sodium hyaluronate content, the purification process should be performed by placing the crosslinked hyaluronic acid, subjected to the crosslinking process, in a tool such as a tube made of a semi-permeable membrane to prevent the crosslinked hyaluronic acid from swelling during the purification process. The purification process using a semi-permeable membrane suppresses the swelling of the crosslinked hyaluronic acid, but has problems in that the process time is long and the operator's convenience is low.

**[Table 1]**

| Comp. Ex. No. | Composition of crosslinking mixture | | | Swelling degree of sample, subjected to only "neutralization process" after crosslinking, in isotonic solution | | |
|---|---|---|---|---|---|---|
| | NaHA (g) | BDDE (g) | 1% NaOH aqueous solution (g) | Swollen weight (g) | Theoretical content (g) of NaHA | Swelling degree (isotonic solution/fold) |
| 1 | 6.000 | 0.090 | 53.910 | 597.2 | 3.0 | 199.1 |
| 2 | | 0.120 | 53.880 | 512.7 | 3.0 | 170.9 |
| 3 | | 0.150 | 53.850 | 462.7 | 3.0 | 154.2 |
| 4 | | 0.090 | 41.910 | 451.2 | 3.0 | 150.4 |
| 5 | | 0.120 | 41.880 | 372.3 | 3.0 | 124.1 |
| 6 | | 0.150 | 41.850 | 296.7 | 3.0 | 98.9 |
| 7 | | 0.090 | 33.910 | 320.1 | 3.0 | 106.7 |
| 8 | | 0.120 | 33.880 | 252.6 | 3.0 | 84.2 |
| 9 | | 0.150 | 33.850 | 214.5 | 3.0 | 71.5 |
| 10 | | 0.090 | 28.196 | 221.4 | 3.0 | 73.8 |
| 11 | | 0.120 | 28.166 | 186.9 | 3.0 | 62.3 |
| 12 | | 0.150 | 28.136 | 166.2 | 3.0 | 55.4 |
| 13 | | 0.090 | 23.910 | 178.8 | 3.0 | 59.6 |
| 14 | | 0.120 | 23.880 | 161.1 | 3.0 | 53.7 |
| 15 | | 0.150 | 23.850 | 152.7 | 3.0 | 50.9 |
| 16 | | 0.090 | 20.577 | 155.4 | 3.0 | 51.8 |
| 17 | | 0.120 | 20.547 | 140.1 | 3.0 | 46.7 |
| 18 | | 0.150 | 20.517 | 128.4 | 3.0 | 42.8 |
| 19 | | 0.090 | 17.910 | 139.4 | 3.0 | 46.5 |
| 20 | | 0.120 | 17.880 | 110.2 | 3.0 | 36.7 |
| 21 | | 0.150 | 17.850 | 96.1 | 3.0 | 32.0 |

On the other hand, Examples 1 to 21, subjected to the "neutralization process" and the "drying process", all showed a swelling degree much lower than 50-fold in the isotonic solution regardless of the hyaluronic acid and 1,4-butanediol diglycidyl ether (BDDE) contents in the crosslinking mixture. In particular, in Examples 1 to 21, the swelling degree of the crosslinked hyaluronic acid in the isotonic solution after drying was 13.7 to 31.5-fold, and in Examples 17 to 21, the swelling degree was 11.9 to 12.4-fold. This means that a crosslinked hyaluronic acid composition having a hyaluronic acid content of 8 wt% or less can be easily produced by drying the crosslinked hyaluronic acid after the crosslinking process.

**[Table 2]**

| Ex. No. | Composition of crosslinking mixture | | | Sample subjected to "neutralization + drying" | | | | | |
|---|---|---|---|---|---|---|---|---|---|
| | | | | After drying | | | After purification | | |
| | NaHA (g) | BDDE (g) | 1% NaOH aqueous solution (g) | Dry weight (g) | Theoretical solid weight (g) | Solid content (%) | Swollen weight (g) | Theoretical NaHA content (g) | Swelling degree (isotonic solution/fold) |
| 1 | 6.000 | 0.090 | 53.910 | 3.426 | 3.180 | 92.8 | 94.5 | 3.0 | 31.5 |
| 2 | | 0.120 | 53.880 | 3.420 | 3.195 | 93.4 | 82.7 | 3.0 | 27.6 |
| 3 | | 0.150 | 53.850 | 3.418 | 3.210 | 93.9 | 71.4 | 3.0 | 23.8 |
| 4 | | 0.090 | 41.910 | 3.376 | 3.255 | 96.4 | 85.2 | 3.0 | 28.4 |
| 5 | | 0.120 | 41.880 | 3.402 | 3.269 | 96.1 | 72.3 | 3.0 | 24.1 |
| 6 | | 0.150 | 41.850 | 3.486 | 3.284 | 94.2 | 66.9 | 3.0 | 22.3 |
| 7 | | 0.090 | 33.910 | 3.370 | 3.215 | 95.4 | 69.3 | 3.0 | 23.1 |
| 8 | | 0.120 | 33.880 | 3.454 | 3.229 | 93.5 | 62.4 | 3.0 | 20.8 |
| 9 | | 0.150 | 33.850 | 3.504 | 3.244 | 92.6 | 58.2 | 3.0 | 19.4 |
| 10 | | 0.090 | 28.196 | 3.315 | 3.186 | 96.1 | 55.2 | 3.0 | 18.4 |
| 11 | | 0.120 | 28.166 | 3.345 | 3.201 | 95.7 | 49.2 | 3.0 | 16.4 |
| 12 | | 0.150 | 28.136 | 3.378 | 3.216 | 95.2 | 42.9 | 3.0 | 14.3 |
| 13 | | 0.090 | 23.910 | 3.388 | 3.165 | 93.4 | 52.2 | 3.0 | 17.4 |
| 14 | | 0.120 | 23.880 | 3.375 | 3.179 | 94.2 | 42.3 | 3.0 | 14.1 |
| 15 | | 0.150 | 23.850 | 3.442 | 3.194 | 92.8 | 38.4 | 3.0 | 12.8 |
| 16 | | 0.090 | 20.577 | 3.286 | 3.148 | 95.8 | 41.1 | 3.0 | 13.7 |
| 17 | | 0.120 | 20.547 | 3.271 | 3.163 | 96.7 | 37.2 | 3.0 | 12.4 |
| 18 | | 0.150 | 20.517 | 3.402 | 3.178 | 93.4 | 35.7 | 3.0 | 11.9 |
| 19 | | 0.090 | 17.910 | 3.246 | 3.067 | 94.5 | 36.4 | 3.0 | 12.1 |
| 20 | | 0.120 | 17.880 | 3.278 | 3.075 | 93.8 | 35.2 | 3.0 | 11.7 |
| 21 | | 0.150 | 17.850 | 3.336 | 3.082 | 92.4 | 35.6 | 3.0 | 11.9 |

### [Examples 22 to 27 and Comparative Examples 22 to 25]

34.3 g of a crosslinking mixture having the same composition as in Example 12 was prepared and subjected to a crosslinking process, a neutralization process, a drying process, and then a purification process to obtain a crosslinked hyaluronic acid (swelling degree in isotonic solution: 14.3-fold). A process of controlling the swelling of the crosslinked hyaluronic acid was performed by adding a 0.01% NaOH aqueous solution in which NaOH is dissolved in physiological saline and physiological saline to 2 g of the obtained crosslinked hyaluronic acid (140 mg as HA), and the total weight of the gel swelling control composition was set to 4 g. The prepared composition containing the crosslinked hyaluronic acid was stored at room temperature (20 to 25°C), and when the added aqueous NaOH solution and physiological saline were completely absorbed into the crosslinked hyaluronic acid, the pH of the swelling control composition was measured using a pH meter, and whether the crosslinked hyaluronic acid maintained its shape was visually checked, thereby completing the swelling control process of controlling the swelling of the crosslinked hyaluronic acid.

After the process of controlling the swelling of the crosslinked hyaluronic acid was completed, an aqueous solution of 40 mmol/kg of sodium phosphate monobasic in which sodium phosphate monobasic is dissolved in physiological saline and physiological saline were added to obtain the final composition. The total weight of the final composition was 7 g, the pH thereof was adjusted to 6 to 8, and the osmotic pressure thereof was adjusted to 300 to 400 mOsmol/kg. It was visually checked whether the solution added to prepare the final mixture was completely absorbed into the crosslinked hyaluronic acid and whether the crosslinked hyaluronic acid maintained its shape.

At this time, Comparative Examples 22 to 25 correspond to cases in which only the "neutralization process" was performed without performing the "drying process", like Comparative Examples 1 to 21.

Composition ratios and measurement results for Examples 22 to 27 and Comparative Examples 22 to 25 are summarized in Table 3 below.

**[Table 3]**

| Sample group | No. | Composition of mixture for controlling swelling of crosslinked hyaluronic acid subjected to purification after drying/results | | | | | | Results of observation after final mixing | |
|---|---|---|---|---|---|---|---|---|---|
| | | Gel weight (g) | HA content (mg) in gel | Amount (g) of aqueous NaOH solution added | Amount (g) of saline added | pH of swelling control mixture | Whether crosslinked hyaluronic acid maintained its shape | Whether added solution was completely absorbed | Whether crosslinked hyaluronic acid maintained its shape |
| Example | 22 | 2.0 | 140 | 0.40 | 1.60 | 11.41 | O | O | O |
| | 23 | | | 0.60 | 1.40 | 11.55 | O | O | O |
| | 24 | | | 0.80 | 1.20 | 11.71 | O | O | O |
| | 25 | | | 1.00 | 1.00 | 11.80 | O | O | O |
| | 26 | | | 1.20 | 0.80 | 11.89 | O | O | O |
| | 27 | | | 1.40 | 0.60 | 11.97 | O | O | O |
| Comp. Example | 22 | 2.0 | 140 | 0.20 | 1.80 | 11.12 | O | X | O |
| | 23 | | | 1.60 | 0.40 | 12.02 | △ | O | △ |
| | 24 | | | 1.80 | 0.20 | 12.06 | △ | O | △ |
| | 25 | | | 2.00 | 0.00 | 12.11 | X | O | X |

Through Examples 22 to 27, it was confirmed that, even if the swelling degree of the crosslinked hyaluronic acid in the isotonic solution was lowered by applying the drying process, when the pH of the swelling control mixture was adjusted to 11.4 to 12.0, the swelling degree could be increased to the desired level while maintaining the shape of the crosslinked hyaluronic acid. The results of Examples 22 to 27 show that no efforts need to be made to devise a hyaluronic acid mixture for crosslinking or control crosslinking conditions to achieve a specific hyaluronic acid content, and the content of the crosslinked hyaluronic acid in the composition containing the crosslinked hyaluronic acid can be freely controlled merely by adjusting the amount of solution added during the swelling control process. On the other hand, it could be seen that, when the pH of the swelling control mixture was lower than 11.4, it was impossible to control the swelling degree of the crosslinked hyaluronic acid, and when the pH of the swelling control mixture was higher than 12.0, it was difficult to maintain the shape of the crosslinked hyaluronic acid.

### [Examples 29 to 31 and Comparative Examples 26 to 29]

34.3 g of a crosslinking mixture having the same composition as in Example 12 was prepared and then subjected to a crosslinking process by storing the same in a constant-temperature bath at 25°C for 24 hours. The crosslinked hyaluronic acid obtained through the crosslinking process was subjected to a neutralization process by immersing the crosslinked hyaluronic acid in 100 g of an aqueous sodium phosphate monobasic solution containing 2.5 wt% of sodium phosphate monobasic so that the aqueous sodium phosphate monobasic solution was completely absorbed into the crosslinked hyaluronic acid. 134.3 g of the crosslinked hyaluronic acid composition obtained through the neutralization process was divided into 20 g portions and subjected to a drying process in a convection oven at 60°C while controlling the drying time. The weight of the dried crosslinked hyaluronic acid composition was measured for each drying time. The water content in the crosslinked hyaluronic acid composition was calculated based on the weight after drying, and the calculated water content and the swelling behavior of the dried crosslinked hyaluronic acid composition in the isotonic solution was measured with the naked eye and by measurement of the swollen weight. The results are summarized in Table 4 below.

At this time, Comparative Examples 26 to 29 corresponds to cases in which only the "neutralization process" was performed without performing the "drying process", like Comparative Examples 1 to 21.

**[Table 4]**

| Sample group | Sample No. | Gel composition after neutralization process | | | Dried gel composition | | | Behavior of dried gel in isotonic solution | | |
|---|---|---|---|---|---|---|---|---|---|---|
| | | Weight (g) of neutralized gel composition | Calculated HA content (g) in neutralized gel composition | Calculated solid content (g) in neutralized gel composition | Drying time (hr) | Weight (g) of dried gel composition | Calculated water content (%) | Appearance | Swollen weight (g) | Swelling degree (fold) |
| Example | 29 | 20.0 | 0.894 | 1.30 | 24 | 1.44 | 9.82 | Uniform | 1.3 | 14.5 |
| | 30 | | | | 26 | 1.40 | 7.24 | Uniform | 12.9 | 14.4 |
| | 31 | | | | 28 | 1.38 | 5.90 | Uniform | 12.8 | 14.3 |
| Comp. Example | 26 | 20.0 | 0.894 | 1.30 | 8 | 10.48 | 88.06 | Non-uniform | 39.4 | 44.1 |
| | 27 | | | | 12 | 6.49 | 8000 | Non-uniform | 34.5 | 38.6 |
| | 28 | | | | 16 | 4.23 | 69.30 | Non-uniform | 29.8 | 33.4 |
| | 29 | | | | 20 | 1.53 | 15.68 | Non-uniform | 16.48 | 18.4 |

As a result of observing the swelling behavior in the isotonic solution for Examples 29 to 31, in which the water content in the dried crosslinked hyaluronic acid was 10 wt% or less, it was confirmed that the crosslinked hyaluronic acid was uniformly swollen and the swelling degree was measured to be 14.3 to 14.5. On the other hand, in Comparative Examples 26 to 29 in which the water content in the dried product was measured to be higher than 15 wt%, it was confirmed that, when the water content was 15 wt% or more, the swelling degree decreased compared to the swelling degree of the undried crosslinked hyaluronic acid (14.3-fold in Example 12), but the swelling behavior in the isotonic solution was non-uniform, indicating that regions with high and low swelling degrees coexisted.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method for producing a composition containing a hyaluronic acid gel-type crosslinked hyaluronic acid produced by chemically modifying hyaluronic acid, the method comprising:
(S1) a pre-crosslinking mixing process of mixing hyaluronic acid or a salt thereof, a chemical crosslinking agent, and a basic substance-containing aqueous solution having a hydrogen ion concentration (pH) of 12 to 14;
(S2) a crosslinking process of forming crosslinks;
(S3) a neutralization process of mixing a crosslinked hyaluronic acid, produced through the crosslinking process, with an aqueous solution containing an acidic substance, thus producing a crosslinked hyaluronic acid having a hydrogen ion concentration (pH) of 9 or less;
(S4) a drying process of removing water from a neutralized mixture prepared through (S3);
(S5) a washing process of removing water-soluble impurities from a hyaluronic acid gel by immersing the dried crosslinked hyaluronic acid, prepared through (S4), in a washing solution, which is an aqueous solution adjusted to an osmotic pressure of 0 to 600 mOsmol/kg using an osmotic pressure adjusting agent; and
(S6) a swelling control process of mixing the crosslinked hyaluronic acid, purified through (S5), with a basic substance or an aqueous solution containing a basic substance and an osmotic pressure adjusting agent.

2. The method according to claim 1, further comprising the following (S7) after (S6):
(S7) a final mixing process of mixing the crosslinked hyaluronic acid, whose swelling has been controlled through (S6), with an acidic substance or an aqueous solution containing an acidic substance, an osmotic pressure adjusting agent, an antioxidant additive, and a pain-relieving additive.

3. The method according to claim 1, wherein (S4) is a drying process of removing water from the neutralized mixture prepared through (S3), thus producing a dried hyaluronic acid gel having a water content of 10% or less of the weight after drying.

4. The method according to claim 1, wherein (S4) comprises a drying process of evaporating water while circulating dry air, and a drying process of removing water by immersing the neutralized crosslinked hyaluronic acid in an organic solvent, and then removing water and the organic solvent while circulating dry air.

5. The method according to claim 4, wherein the organic solvent is at least one selected from the group consisting of methanol, ethanol, propanol, 1,2-butanediol, 1,3-butanediol, 1,5-pentanediol, 1,2-hexanediol, acetone, acetaldehyde, acetonitrile, tetrahydrofuran, glycerin, and tetraethylene glycol.

6. The method according to claim 1, wherein the crosslinking agent is at least one selected from the group consisting of 1,4-butandiol diglycidyl ether, ethylene glycol diglycidyl ether, 1,6-hexanediol diglycidyl ether, propylene glycol diglycidyl ether, polyethylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, trimethlypropane polyglycidyl ether, 1,2-bis(2,3-epoxypropoxy)ethylene, pentaerythritol polyglycidyl ether, and sorbitol polyglycidyl ether.

7. The method according to claim 1, wherein the basic substance in (S1) and (S6) is at least one selected from the group consisting of lithium hydroxide, sodium hydroxide, potassium hydroxide, calcium hydroxide, and aluminum hydroxide.

8. The method according to claim 1 or 2, wherein the acidic substance comprises at least one selected from the group consisting of an aqueous phosphoric acid solution, an aqueous acetic acid solution, an aqueous hydrochloric acid solution, an aqueous nitric acid solution, an aqueous sulfuric acid solution, an aqueous boric acid solution, an aqueous diethyl barbituric acid solution, sodium phosphate monobasic or sodium phosphate monobasic hydrate, potassium phosphate monobasic or potassium phosphate monobasic hydrate, ammonium phosphate monobasic or ammonium phosphate monobasic hydrate, and sodium acetate or sodium acetate hydrate.

9. The method according to claim 1, wherein (S6) is a swelling control process of mixing the crosslinked hyaluronic acid, purified through (S5), with a basic aqueous solution so that the hydrogen ion concentration (pH) is adjusted 11.4 to 12.0.

10. The method according to claim 2, wherein (S7) is a final mixing process of mixing the crosslinked hyaluronic acid, whose physical properties have been controlled through (S6), with an acidic substance or an aqueous solution containing an acidic substance, an osmotic pressure adjusting agent, a viscosity-controlling additive, and a pain-relieving additive, thus producing a composition having a hydrogen ion concentration (pH) of 5 to 9 and a hyaluronic acid content of 0.5 to 5 wt%.

11. The method according to claim 1 or 2, wherein the osmotic pressure adjusting agent is at least one selected from the group consisting of sodium chloride (NaCl), potassium chloride (KCl), calcium chloride (CaCl₂), and magnesium chloride (MgCl₂).

12. The method according to claim 2, wherein the pain-relieving additive is any one or more selected from the group consisting of lidocaine or a salt thereof and bupivacaine or a salt thereof.

13. The method according to claim 2, wherein the antioxidant additive is at least one selected from the group consisting of ascorbic acid or a derivative thereof, glutathione, lipoic acid or a derivative thereof, uric acid or a derivative thereof, tocopherol, and mannitol.
